(19) Europäisches Patentamt / European Patent Office / Office européen des brevets

(11) **EP 4 790 109 A1**

(12) **EUROPEAN PATENT APPLICATION**
published in accordance with Art. 153(4) EPC


(43) Date of publication:
**12.08.2026 Bulletin 2026/33**

(21) Application number: **24907113.5**

(22) Date of filing: **28.11.2024**

(51) International Patent Classification (IPC):
***C09B 67/02*** *(2006.01)* ***A61K 8/25*** *(2006.01)*
***A61K 8/26*** *(2006.01)* ***A61Q 1/00*** *(2006.01)*

(52) Cooperative Patent Classification (CPC):
**A61K 8/25; A61K 8/26; A61Q 1/00; C09B 67/0097**

(86) International application number:
**PCT/JP2024/042193**

(87) International publication number:
**WO 2025/134726 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.12.2023 JP 2023213955**



(71) Applicant: **DIC Corporation**
**Tokyo 174-8520 (JP)**

(72) Inventors:
- **MIKAMI, Kaiyu**
  **Kamisu-shi, Ibaraki 314-0193 (JP)**
- **SUGIURA, Kenichi**
  **Kamisu-shi, Ibaraki 314-0193 (JP)**
- **GUNJI, Yasuhiko**
  **Kamisu-shi, Ibaraki 314-0193 (JP)**

(74) Representative: **TBK**
**Bavariaring 4-6**
**80336 München (DE)**


(54) **COMPOSITE PIGMENT AND METHOD FOR PRODUCING SAME**


(57) A composite pigment including a base material and a pigment coating a surface of the base material is provided. The pigment includes an organic pigment. When 0.05 g of the composite pigment is dispersed in 20 mL of ethyl acetate and a resulting dispersion is allowed to stand for 24 hours and separated into a precipitate and a supernatant, the supernatant has an absorbance of 0.15 or less at a maximum absorption wavelength.


FIG. 1

1
2
3
4
5
6
7
8
9


EP 4 790 109 A1

## Description

Technical Field

The present disclosure relates to a composite pigment and a method for producing the same.

Background Art

Traditionally, in the fields of cosmetics, inks, and paints, there is a demand for materials that exhibit good color development and gloss when applied, and have excellent brightness. In particular, make-up cosmetics such as lipsticks, eye shadows, blushers, and nail polishes require to exhibit uniform color development in order to provide a better finish when applied to the skin. Under these circumstances, a composite pigment formed by compounding a base material (e.g., a sparkling material such as mica) and a pigment (e.g., an organic pigment) has been used in the fields of cosmetics, inks, paints, and the like.

For example, PTL 1 describes a flaky pigment having a mean particle size of 5 to 60 μm, which is in the form of composite particles based on an ordered mixture, wherein the composite particles are formed by performing a high-speed stirring process on a mixture of a flaky substrate material made of flaky substrate particles having an aspect ratio of 10 to 120 and a pigment and/or a dye made of particles having a mean particle size of 5 μm or less in the absence of a liquid medium.

In recent years, dry particle compounding devices have been used to compound a base material and a pigment.

For example, PTL 2 discloses a processing device including a rotating shaft having a plurality of stirring members on its outer circumference, and a casing having an inner circumference positioned with a small gap from the stirring members, the processing device performing a stirring process on a material to be processed in the casing with the stirring members moving as the rotating shaft rotates, wherein when viewed from a direction orthogonal to an axial direction of the rotating shaft, an end position of each of the stirring members in a direction parallel to the axial direction of the rotating shaft is located inside adjacent another stirring member relative to an end position of the other stirring member. PTL 2 also discloses a processing device including a rotating shaft having a plurality of stirring members on its outer circumference, and a casing having an inner circumference positioned with a small gap from the stirring members, the processing device performing a stirring process on a material to be processed in the casing with the stirring members moving as the rotating shaft rotates, wherein the inner circumference of the casing has a diameter twice or less the diameter of the outer circumference of the rotating shaft.

The device described in PTL 2 has a structure, for example, illustrated FIGS. 1 to 3. The device having the structure illustrated in FIGS. 1 to 3 is described in PTL 2 as follows.

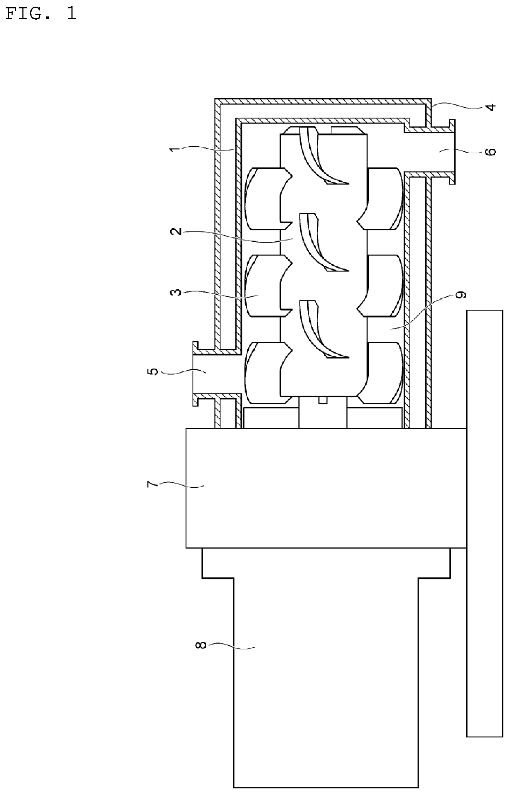

"The device has a rotating shaft 2 with a plurality of stirring members 3 on its outer circumference at the center of a cylindrical casing 1 encased in a jacket 4. The casing 1 has an inner circumference positioned with a small gap (clearance) from the stirring members 3. A stirring process is performed on a material to be processed in the casing 1 with the stirring members 3 moving as the rotating shaft 2 rotates. The rotating shaft 2 is supported on one side by a bearing section 7 and is connected to a driver section 8 including a motor and the like. A material feed port 5 is provided at an end side surface or at the top of the casing 1, and a product discharge port 6 is provided at the bottom of the casing 1 at an end opposite to the powder feed port 5. That is, the rotating shaft 2 is supported only at one end (left side in FIG. 1) in the axial direction, and the casing 1 is formed into a bottomed cylindrical shape that is open only at one end (left side in FIG. 1) in the axial direction of the rotating shaft 2 and closed at the other end (right side in FIG. 1), and is movable along the axial direction of the rotating shaft 2 between an operating position (position in FIG. 1) where a processing space 9 between the casing 1 and the rotating shaft 2 is covered and a non-operating position (not illustrated) where the processing space 9 is not covered." (paragraphs [0039], [0040])

"The outer circumference of the rotating shaft 2 has plate-shaped stirring members 3a, 3b at an angle inclined relative to the axial direction, and both end faces 2a of the rotating shaft 2 have diffusing members 10." (paragraph [0043])

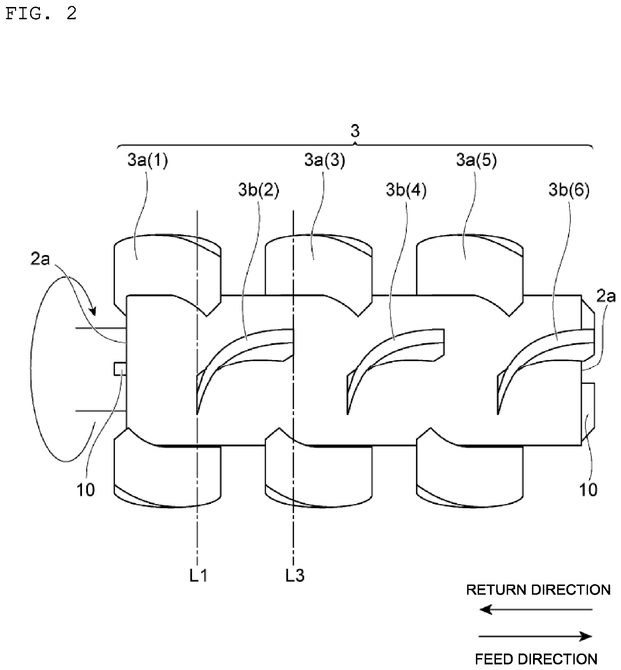

"As illustrated in FIG. 2, when the rotating shaft 2 of the device is viewed from a position orthogonal to the axial direction, for example, the end position of the stirring member 3b(2) in the direction parallel to the axial direction of the rotating shaft 2 is located inside adjacent other stirring members 3a(1), 3a(3) relative to the end positions of the other stirring members 3a(1), 3a(3). In other words, if extensions L1, L3 are drawn vertically from the end portions of the stirring member 3b(2), the end portions of the stirring member 3b(2) are in a positional relation so as to overlap parts of the adjacent stirring members 3a(1), 3a(3). The other stirring members 3a(1), 3a(3), 3b(4), 3a(5), 3b(6) are in the same positional relation. When the stirring members 3a, 3b are in such a positional relation, powder enters from the end portions of the stirring members 3a, 3b deeply to the inside of the adjacent other stirring members 3a, 3b. As a result, the force of the stirring members can be strongly transmitted to the powder." (paragraph [0044])

"In the present device, as illustrated in FIG. 3, the diameter D1 of the inner circumference of the casing 1 is twice or less the diameter D2 of the outer circumference of the rotating shaft 2. In other words, their relation can be expressed by

$D1 \leq D2 \times 2$. FIG. 3 illustrates an example in which D1 is 1.8 times larger than D2. By making D2 relatively large relative to D1, the space (processing space) 9 in which the force acts on the powder is limited, and as a result, the force of the stirring members 3a, 3b can be strongly transmitted to the powder even with the same peripheral speed of the stirring members 3a, 3b. If D1 exceeds twice D2, the space 9 in which the force acts on the material to be processed becomes too large, resulting in smaller force exerted on the powder." (paragraph [0045])

"At least one or more of the stirring members 3a, 3b are formed as feed stirring members 3a that feed the material to be processed in one direction of the axial direction of the rotating shaft 2 as the rotating shaft 2 rotates, and one or more other stirring members 3a, 3b are formed as return stirring members 3b that return the material to be processed in the other direction of the axial direction of the rotating shaft 2 as the rotating shaft 2 rotates." (paragraph [0046])

"As illustrated in FIG. 2, the plate surface of the feed stirring member 3a is inclined to feed the powder in the feed direction, that is, in one direction of the axial direction of the rotating shaft 2 as the rotating shaft 2 rotates. When the material feed port 5 and the product discharge port 6 are provided at both ends of the casing 1 (in the case of FIG. 1), the direction from the material feed port 5 to the powder discharge port 6 (right direction in FIG. 2) is referred to as feed direction. On the other hand, the plate surface of the return stirring member 3b is inclined to return the powder in the return direction, that is, in the opposite direction of the feed direction of the axial direction of the rotating shaft 2 as the rotating shaft 2 rotates. When the material feed port 5 and the product discharge port 6 are provided at both ends of the casing 1 (in the case of FIG. 1), the direction from the product discharge port 6 to the material feed port 5 (left direction in FIG. 2) is referred to as return direction. The inclination angle of the stirring members 3a, 3b is preferably set within the range of $\pm 5$ to $\pm 85$ degrees relative to the axial direction of the rotating shaft 2." (paragraph [0047])

"Among the stirring members 3a, 3b, a plurality of members disposed at intervals in the circumferential direction of the rotating shaft 2 are formed as a set. The letters (1), (2), (3), and the like appended after 3a, 3b distinguish each set of the stirring members 3a, 3b. In FIG. 2, the stirring members 3a, 3b in the same set are inclined relative to the rotating shaft 2 to guide the powder in the same direction, that is, either in the feed or return direction. However, embodiments are not limited to this. In FIG. 2, among the stirring members 3a, 3b, two members at 180 degree apart from each other on the rotating shaft 2 form a set. However, a number of members may form a set, such as three at 120 degree interval or four at 90 degree interval." (paragraph [0048])

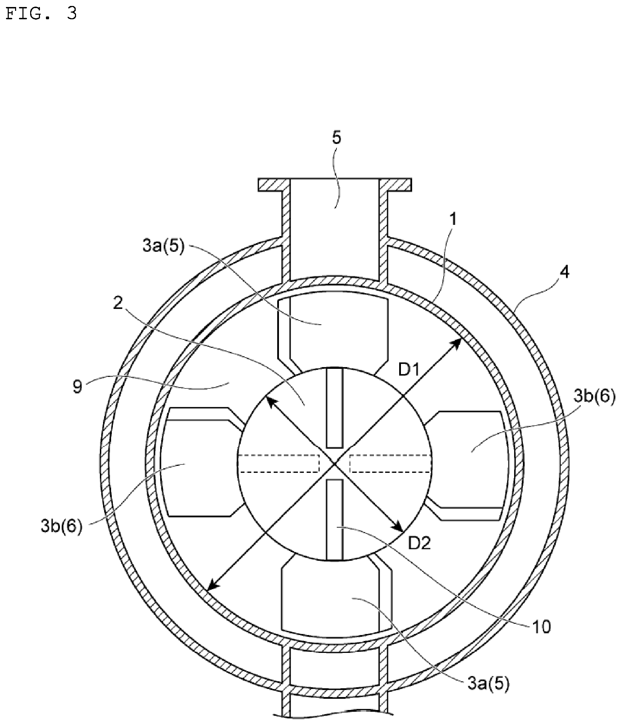

"One of the stirring members 3a, 3b adjacent to each other in the circumferential direction of the rotating shaft 2 is formed as the feed stirring member 3a, and the other 3b is formed as the return stirring member 3b. Specifically, as illustrated in FIG. 3, one 3a(5) of the stirring members 3a(5), 3b(6) adjacent to each other in the circumferential direction of the rotating shaft 2 is formed as the feed stirring member 3a, and the other 3b(6) is formed as the return stirring member 3b. It is desirable that each set of stirring members 3a, 3b is offset at a certain angle from another set of stirring members 3a, 3b adjacent to each other in the axial direction of the rotating shaft 2, as viewed from the direction of the rotating shaft. In FIG. 3, the offset is 90 degrees, but is not limited to this angle." (paragraph [0049])

"In FIG. 2, three sets of feed stirring members 3a and return stirring members 3b are provided alternately in the axial direction of the rotating shaft 2, and in total, six sets are provided. In this case, the powder alternately receives the force of "feed, return, feed, return, feed, return," which makes the movement path of the powder in the casing more complicated and longer than when the powder receives the force in only one direction. As a result, the powder receives even stronger force of the stirring members 3a, 3b." (paragraph [0050])

"In FIG. 2 ..., the inclination angles of the feed stirring members 3a ... and the return stirring members 3b ... are the same, but are not limited to this. All of the stirring members 3a, 3b may have different inclination angles or only some may have different inclination angles." (paragraph [0051])

"The stirring members 3a, 3b are formed in the shape of plates. Since the stirring members 3a, 3b are formed in the shape of plates, the entire rotating shaft 2 can be lighter in weight than when it has block-shaped stirring members, which makes it possible to design the rotating shaft 2 to rotate at a higher speed. As a result, the force of the stirring members 3a, 3b can be transmitted even more strongly to the powder. The "shape of plate" is intended to mean that it is sufficient if at least the portions of the stirring members 3a, 3b that are close to the inner circumference of the casing 1 are formed in the shape of plates, and the rotating shaft 2 and the stirring members 3a, 3b may be joined by rod-shaped arms or the like." (paragraph [0052])

"It is desirable that the gap (clearance) between the inner circumference of the casing 1 and the stirring members 3 is kept constant and small. The reason for keeping the clearance constant is to apply force evenly to the powder, while the reason for keeping the clearance small is to apply force more strongly by reducing the escape of the powder. If simple rectangular plate-shaped members as viewed from the direction crossing the axial direction are provided as the stirring members 3a, 3b on the rotating shaft 2, the clearance between the central portions of the plate-shaped members and the inner circumference of the casing 1 is wider than those at both ends of the plate-shaped members. It is therefore desirable that the shape of the stirring members 3a, 3b on the inner circumference side of the casing 1 is designed so that the clearance is kept constant. However, the clearance between the inner circumference of the casing 1 and one set of stirring members 3 and the clearance between the inner circumference and another set of stirring members 3 need not be the same. For example, in FIG. 2, the clearance of the stirring member 3a(1) at a feed proximal end closer to the material feed

port 5 may be wider than the clearance of the stirring member 3a(5) at a feed distal end closer to the product discharge port 6. It is preferable that the clearance is in the range of 0.05 to 7.5% of the diameter D1 of the casing inner circumference, desirably 0.75 to 3%. If the clearance exceeds 7.5%, the escape of the powder becomes large, which prevents application of strong force. If the clearance is 0.05% or less, there is a risk of contact between the stirring members 3a, 3b and the casing 1 due to vibrations that occur during operation. To be specific, it is desirable to set the gap between the inner circumference of the casing 1 and the stirring members 3a, 3b in the range of 0.3 mm to 50 mm." (paragraphs [0053], [0054])

"To ensure that the material to be processed can receive effective stirring action in the casing 1, it is desirable that the input volume of the material to be processed relative to 100% of the internal volume of the processing space 9 in the space casing 1 is set in the range from a lower limit of 5% to an upper limit of 95%. Here, the internal volume of the processing space 9 in the casing 1 means the volume of the space obtained by subtracting the volume occupied by the rotating shaft 2 from the internal volume of the casing 1 itself (the substantial space in which the material to be processed can move around in the casing 1)." (paragraph [0055])

"Both ends of the rotating shaft 2 are sections where the action of the stirring members 3a, 3b is difficult to reach. Therefore, the feed stirring member 3a is provided at one end of the rotating shaft 2 and the return stirring member 3b is provided at the other end to suppress movement of the powder to both ends of the rotating shaft 2, thereby preventing the powder from being discharged without receiving strong stirring action by the stirring members 3. That is, the stirring member 3a(1) located at one end (left end in FIG. 2 ...) of the axial direction of the rotating shaft 2 is formed as the feed stirring member 3a that feeds the material to be processed from one end to the other end (right end in FIG. 2 ...) of the axial direction, and the stirring members 3b(5), 3b(6) located at the other end (right end in FIG. 2 ...) of the axial direction of the rotating shaft 2 are formed as the return stirring members 3b that return the material to be processed from the other end to the one end of the axial direction." (paragraph [0057])

"The stirring members 3a, 3b are actually ... disposed such that the distal end portions of the stirring members 3a, 3b facing the inner circumference of the casing 1 are formed in an acute angle shape in cross-sectional view when viewed from the axial direction of the rotating shaft 2, and the center line L of the distal-end acute angle portion is inclined from a right angle direction relative to the inner circumferential surface of the casing 1. ... the angle of the distal-end acute angle portion is not limited to 60 degrees, but can be set to any angle from an angle close to 90 degrees to less than 60 degrees. However, considering the wear of the distal end portion, too small an angle is not preferred. The inclination angle of the center line L can also be set to any appropriate value." (paragraph [0058])

"The diffusing members 10 are provided on both end faces 2a of the rotating shaft 2. As the diffusing members 10 move with the rotation of the rotating shaft 2, the diffusing members 10 generate centrifugal force for the powder that attempts to intrude toward both end faces 2a where the action of the stirring members 3 is difficult to reach, thereby suppressing the movement of the powder toward both end faces 2a. This prevents the powder from being discharged without receiving strong stirring action by the stirring members 3. As illustrated in FIG. 3, the diffusing members 10 are each include two plate-shaped members extending in opposite directions from the center of the rotating shaft 2 at each end face 2a. As indicated by solid and dashed lines, the diffusing members 10 on both end faces 2a are disposed to be offset 90 degrees from each other as viewed in the axial direction of the rotating shaft 2. However, the configuration is not limited to this, and other shapes, angles, and numbers of diffusing members may be employed as long as they can generate centrifugal force, and the diffusing member may cross the center of the rotating shaft 2 when viewed from the axial direction of the rotating shaft 2. Although the diffusing member 10 may be provided only on one end face 2a of the rotating shaft 2, it is desirable that the diffusing members 10 are provided on both end faces 2a." (paragraph [0060])

Citation List

Patent Literature

PTL 1: Japanese Unexamined Patent Application Publication No. H05-214257
PTL 2: Japanese Unexamined Patent Application Publication No. 2005-270955

Summary of Invention

Technical Problem

One of skin problems caused by common cosmetics is pigmentation on the skin. On the other hand, a composite pigment having a base material coated with an organic pigment has the problem of causing pigmentation on the skin when used as a cosmetic.

An object according to some aspects of the present disclosure is to provide a composite pigment less likely to cause pigmentation on the skin when used as a cosmetic and a method for producing the same.

Solution to Problem

The inventors of the present disclosure have found that a composite pigment with specific bleeding performance can solve the above problem. This finding has led to the present disclosure.

Some aspects of the present disclosure provide the following [1] to [7].

[1] A composite pigment including a base material and a pigment coating a surface of the base material, wherein

the pigment includes an organic pigment, and
when 0.05 g of the composite pigment is dispersed in 20 mL of ethyl acetate and a resulting dispersion is allowed to stand for 24 hours and separated into a precipitate and a supernatant, the supernatant has an absorbance of 0.15 or less at a maximum absorption wavelength.

[2] The composite pigment according to [1], wherein the composite pigment has a strong coverage ratio of 80% or more.
[3] The composite pigment according to [1] or [2], wherein the base material and the pigment are compounded by mechanochemical treatment.
[4] The composite pigment according to any one of [1] to [3], wherein the base material is a flaky base material including at least one selected from the group consisting of mica, aluminum, alumina, and glass.
[5] The composite pigment according to any one of [1] to [4], wherein

the base material includes a core and a shell coating a surface of the core, and
the shell includes at least one selected from the group consisting of resins, metals, and metal oxides.

[6] A method for producing a composite pigment including a base material and a pigment coating a surface of the base material,

the method including a coating step of coating the surface of the base material with the pigment by compounding the base material and the pigment including an organic pigment using a dry particle compounding device, wherein when the amount of load power consumed by the dry particle compounding device in the coating step is P1, the amount of load power consumed by the dry particle compounding device when the coating step is performed without a raw material is P2, and the mass of the raw material used in the coating step is m, (P1-P2)/m is 0.2 to 0.5 W·h/g.

[7] The method for producing a composite pigment according to [6], wherein in a volume-based particle size distribution of the pigment measured by a wet laser diffraction-scattering method, when particle sizes at which cumulative values from smaller diameters reach 10% and 90% of the total volume are D10 and D90, respectively, D90-D10 is 300 μm or less.

Advantageous Effects of Invention

According to the present disclosure, a composite pigment less likely to cause pigmentation on the skin when used as a cosmetic and a method for producing the same can be provided.

Brief Description of Drawings

FIG. 1 is a front partial cross-sectional view of a structure of a processing device according to the invention described in PTL 2.
FIG. 2 is a diagram illustrating a rotating shaft and stirring members in the processing device of FIG. 1.
FIG. 3 is a side cross-sectional view of the structure of the processing device according to the invention described in PTL 2.

Description of Embodiments

Herein, a numerical range indicated by using "to" indicates a range that includes the numerical values listed before and after "to" as the minimum and maximum values. The units of the numerical values listed before and after "to" are the same, unless explicitly specified otherwise. In a numerical range described herein, the upper or lower limit of the numerical range may be replaced by the values listed in the examples. The upper and lower limits listed individually can be combined as desired. The term "A or B" may include either A or B, or both.

Exemplary embodiments of the present disclosure will be described below. However, the present disclosure is not limited in any way to the following embodiments.

<Composite Pigment>

One embodiment of the present disclosure is a composite pigment including a base material and a pigment coating a surface of the base material (hereinafter also referred to as "coating pigment"). The coating pigment includes an organic pigment. When 0.05 g of the composite pigment is dispersed in 20 mL of ethyl acetate and the resulting dispersion is then allowed to stand for 24 hours and separated into a precipitate and a supernatant, the absorbance of the supernatant at the maximum absorption wavelength (hereinafter referred to as "absorbance A") is 0.15 or less.

The composite pigment having the above characteristics is less likely to cause pigmentation on the skin when used as a cosmetic. In other words, the composite pigment having the above characteristics has excellent pigmentation resistance. The composite pigment described above is therefore suitably used as a pigment for cosmetics. The pigmentation resistance of the composite pigment can be evaluated by the following method.

The composite pigment (about 50 mg) is applied and spread with a finger on a 5 mm × 5 mm section of a BIOSKIN plate 100 × 100 mm urethane No. 132 series (No.132#W manufactured by Beaulax Co., Ltd.). Excess pigment is lightly blown off with air, and an appropriate amount (about 0.1 g) of cleansing oil (e.g., Deve OLIVE & ARGAN manufactured by BEAUA PRODUCTS Co., Ltd.) is put on the pigment-applied area. The pigment-applied area is rubbed with a finger for two seconds, the oil is wiped off with KimWipes, and the pigment-applied area is wiped up. After wiping up, the pigment-applied area is colorimetrically measured with a colorimeter (e.g., eXact Advanced (x-Rite)) (illuminant: D50, standard observer: 2°). In the colorimetric data obtained, the smaller the C* value, the more stainless and the better pigmentation resistance.

The C* value of the composite pigment as measured by the above method is, for example, 3.0 or less and can be as low as 0.0. That is, the C* value of the composite pigment as measured by the above method may be, for example, 0.0 to 3.0 or 0.9 to 3.0.

The reason for the above effect is not clear, but is inferred as follows.

First, a possible cause of pigmentation on the skin when a composite pigment is used as a cosmetic is a bleeding phenomenon in which a pigment component (organic pigment, etc.) bleeds out from the composite pigment. In this respect, the absorbance A indirectly indicates the amount of pigment component (organic pigment, etc.) that bleeds from the composite pigment into the dispersion, and it can be said that the lower the absorbance A, the less likely the composite pigment is to cause bleeding and the better the bleed resistance. In this respect, since the above composite pigment has an absorbance A of 0.15 or less, it is inferred that the composite pigment has good bleed resistance and, as a result, is less likely to cause pigmentation on the skin when used as a cosmetic.

The absorbance A can be measured using a spectrophotometer (e.g., U-3900 manufactured by Hitachi, Ltd.). The absorbance A may be 0.10 or less, 0.06 or less, or 0.03 or less from the viewpoint of enhancing the above effect. The lower limit of the absorbance A is not limited, but may be 0.00 or 0.01. That is, the absorbance A may be, for example, 0.00 to 0.15, 0.01 to 0.10, 0.01 to 0.06, or 0.01 to 0.03.

The composite pigment may be a so-called core-shell particle. That is, the composite pigment may have a core formed of a parent particle and a shell formed of a secondary particle. In the composite pigment, the base material may form the core as a parent particle and the coating pigment may form the shell as a secondary particle.

In the composite pigment, the base material and the coating pigment may be compounded by mechanochemical treatment. Here, the mechanochemical treatment refers to a treatment that can induce a mechanochemical phenomenon between the base material and the coating pigment. For example, a treatment using a dry particle compounding device is mechanochemical treatment. The mechanochemical phenomenon is a phenomenon caused by mechanical energy (e.g., compression, shear, impact, etc.) and includes chemical reactions such as formation and breakage of chemical bonds (mechanochemical reaction) and activation caused by particle miniaturization and changes in crystal structure (mechanical activation).

When the base material and the coating pigment are compounded by mechanochemical treatment, the coating pigment is physically fixed and pressed to the surface of the base material by the mechanochemical phenomenon, and the base material and the coating pigment are strongly bonded compared to when the coating pigment simply adheres to the base material. Therefore, such a composite pigment is less likely to cause bleeding of the pigment component described above, and pigmentation on the skin can be suppressed more when the composite pigment is used as a cosmetic.

The composite pigment may have a strong coverage ratio of 80% or more or may have a strong coverage ratio of 87% or more. The higher the strong coverage ratio of the composite pigment, the less likely the composite pigment is to cause bleeding of the pigment components described above, and the more the pigmentation on the skin can be suppressed when used as a cosmetic. The upper limit of the strong coverage ratio of the composite pigment is not limited, but may be, for example, 95%. That is, the strong coverage ratio of the composite pigment may be, for example, 80 to 95% or 87 to 95%. Here, the strong coverage ratio indicates the percentage of the coating pigment that is strongly fixed onto the base material in the composite pigment. The higher the strong coverage ratio, the more coating pigment is strongly bonded to the base material.

The strong coverage ratio can be calculated based on the following formula (a) by measuring absorbance A1 and absorbance A2 according to conditions 1 and 2 below.

strong coverage ratio [unit:%]=(1-[absorbance A1]/[absorbance A2])×100     Formula (a) :

[Condition 1]

After 0.05 g of the composite pigment is weighed in a 30 mL vial, 20 mL of a 5% aqueous ethanol solution is added to the vial. The liquid in the vial is then shaken and dispersed with a paint conditioner (Toyo Seiki Seisaku-sho, Ltd.) for one minute. The vial is then allowed to stand, and 1 mL of the resulting supernatant is sampled and diluted with 9 mL of a diluting solvent to obtain a diluent. The absorbance of the prepared diluent is measured using a spectrophotometer (e.g., U-3900 manufactured by Hitachi, Ltd.), and the absorbance at the maximum absorption wavelength obtained is determined (absorbance A1).

[Condition 2]

After 0.05 g of the composite pigment is weighed in a 30 mL vial, 20 mL of a diluting solvent is added to the vial. The liquid in the vial is then shaken and dispersed with a paint conditioner (Toyo Seiki Seisaku-sho, Ltd.) for one minute. The vial is then allowed to stand, and 1 mL of the resulting supernatant is sampled and diluted with 9 mL of a diluting solvent to obtain a diluent. The absorbance of the prepared diluent is measured using a spectrophotometer (e.g., U-3900 manufactured by Hitachi, Ltd.), and the absorbance at the maximum absorption wavelength obtained is determined (absorbance A2).

The diluting solvent in conditions 1 and 2 is a solvent capable of completely dissolving 0.005 g of the organic pigment in a volume of 20 mL. As used herein "completely dissolving" means that the amount of residue is 0.15 mg or less when 0.005 g of the organic pigment is added to 20 mL of the solvent and stirred well, and the resulting mixture is filtered through a membrane filter with a pore diameter of 0.45 μm (for example, DISMIC-13HP ADVANTEC TOYO KAISHA, LTD). As the diluting solvent, for example, the solvents listed in "Determination Methods" in "Handbook of Legal Pigments, Revised Edition, Japan Cosmetic Industry Association" can be used for the organic pigment contained in the coating pigment. If the organic pigment contained in the coating pigment is Red No. 202, ethanol (acidic dilution) may be used. If the organic pigment contained in the coating pigment is Red No. 104 aluminum lake, a sodium hydroxide reagent (dilution) may be used. If the organic pigment contained in the coating pigment is Blue No. 1 aluminum lake, a sodium hydroxide reagent (dilution) may be used.

The maximum absorption wavelength in conditions 1 and 2 is the maximum absorption wavelength of the organic pigment corresponding to the diluting solvent. If the organic pigment contained in the coating pigment consists of only one type of pigment listed in "Handbook of Legal Pigments, Revised Edition, Japan Cosmetic Industry Association", the maximum absorption wavelength listed in "Determination Methods" in the above handbook for the organic pigment is the maximum absorption wavelength in conditions 1 and 2. For example, if the organic pigment contained in the coating pigment consists only of Red No. 202, the maximum absorption wavelength is 521 nm. If the organic pigment contained in the coating pigment consists only of Red No. 104 aluminum lake, the maximum absorption wavelength is 538 nm. If the organic pigment contained in the coating pigment consists only of Blue No. 1 aluminum lake, the maximum absorption wavelength is 630 nm.

If the coating pigment includes different types of organic pigments, the strong coverage ratio for each organic pigment is determined, and the one with the lowest value among those strong coverage ratios is determined as the strong coverage ratio of the composite pigment. For example, if the coating pigment includes two types of organic pigments (organic pigments A and B), first, a diluting solvent for each of organic pigments A and B is selected. Then, absorbance A1 and A2 are measured using the diluting solvent of organic pigment A as the diluting solvent in conditions 1 and 2, and the strong coverage ratio for organic pigment A is determined. Similarly, absorbance A1 and A2 are measured using the diluting solvent of organic pigment B as the diluting solvent in conditions 1 and 2, and the strong coverage ratio for organic pigment B is determined. The one with the lower value of the thus obtained strong coverage ratios is determined as the strong coverage ratio of the composite pigment.

The constituent materials of the composite pigment will now be described.

(Base Material)

The base material is, for example, an inorganic base material formed of an inorganic material. The inorganic base material may include at least one metal or metal oxide. Examples of the metal include silicon (Si), iron (Fe), aluminum (Al), sodium (Na), calcium (Ca), magnesium (Mg), potassium (K), copper (Cu), manganese (Mn), titanium (Ti), silver (Ag), gold (Au), platinum (Pt), lead (Pb), chromium (Cr), tin (Sn), molybdenum (Mo), gallium (Ga), and indium (In). Examples of the metal oxide include oxides of these metals. The base material may contain an organic material as long as the effects of the present disclosure are not impaired.

The base material is, for example, a particle. The shape of the base material is not particularly limited, but may be flaky (flake-like) from the viewpoint of easy binding to the coating pigment. Examples of the flaky base material include mica, aluminum, alumina, glass, titanium dioxide, iron red, iron oxide, yellow iron oxide, black iron oxide, zinc oxide, iron blue, ultramarine blue, chromium oxide, kaolin, clay, bentonite, bismuth oxychloride, zirconium oxide, and magnesium oxide. These base materials may be used alone or in combination of two or more. Among the above, at least one selected from the group consisting of mica, aluminum, alumina, and glass may be used from the viewpoint of enhancing sparklingness required for cosmetics.

Examples of the mica include natural mica, synthetic mica, synthetic phlogopite, mica titanium, synthetic mica titanium, iron oxide coated mica, iron oxide coated synthetic mica, and chromium hydroxide mica. Commercially available products such as SunMICA (trademark, manufactured by Sun Chemical) and IRIODIN (registered trademark, manufactured by Merck KGaA) can be used.

Examples of the aluminum (Al) include aluminum alone, aluminum hydroxide, aluminum chloride, aluminum nitride, aluminum phosphate, and aluminum sulfate. Commercially available products such as HYDROLAN (registered trademark, manufactured by Eckart GmbH) can be used.

The glass may be silicate glass mainly composed of silicon dioxide ($SiO_2$). Examples of the glass include soda lime glass, lead glass, soda lime glass, A-glass, C-glass, E-glass, borosilicate glass, and aluminosilicate glass. Commercially available products such as GLASFLAKE, METASHINE, and LUMIGLAN (all of the above are registered trademarks, manufactured by Nippon Sheet Glass Co., Ltd.) can be used.

The base material may be a core/shell particle including a core (substrate) and a shell (coating layer) coating the surface of the core. The core may be formed of any of the materials described above. The core may be, for example, flaky. The flaky core may include at least one selected from the group consisting of mica, aluminum, alumina, and glass from the viewpoint of enhancing sparklingness required for cosmetics. The shell may include at least one selected from the group consisting of resins, metals, and metal oxides, as a component. From the viewpoint of enhancing sparklingness required for cosmetics, the base material may have a flaky core and a shell that coats the surface of the core and includes at least one selected from the group consisting of resins, metals and metal oxides.

Examples of the resins include acrylic resins, urethane resins, styrene resins, ether resins, and epoxy resins. Examples of the metals and the metal oxides include the same metals and metal oxides described above.

The core and the shell may be formed of different materials from each other from the viewpoint of enhancing sparklingness required for cosmetics. In particular, the core may include mica and the shell may include titanium oxide from the viewpoint of enhancing sparklingness required for cosmetics. An example of the base material having such a structure is a pearlescent pigment. The hue of the pearlescent pigment is not particularly limited.

The size of the base material may be larger than the coating pigment (one particle). The average particle size of the base material may be 1 to 80 μm, 5 to 50 μm, or 10 to 45 μm from the viewpoint of treatment efficiency of the coating pigment on the base material during composite pigment preparation. Here, the average particle size of the base material means the particle size (median size D50) at which the cumulative value from smaller diameters reaches 50% of the total volume in the volume-based particle size distribution of the base material measured by a wet laser diffraction-scattering method. The average particle size is measured, for example, using a particle size distribution analyzer MT3000II (MicrotracBEL Corp.).

When the base material is flaky, the thickness of the base material may be, for example, 0.1 to 5 μm and may be 0.2 to 3 μm. The length in the longitudinal direction of the flaky base material is, for example, 3 to 100 μm or may be 5 to 50 μm. The average aspect ratio (length/thickness) of the flaky base material is, for example, 10 to 200 or may be 20 to 150. When the size and the average aspect ratio of the flaky base material are within the above ranges, the composite pigment, when used in a cosmetic, tends to provide a smooth feel of a coating of the cosmetic, spread well when applied, and exhibit good color development and gloss.

The content of the base material may be 20 to 98% by mass or 30 to 95% by mass with respect to the total amount of the composite pigment. When the content of the base material is within the above range, the composite pigment, when used in a cosmetic, tends to provide a smooth feel of a coating of the cosmetic, spread well when applied, and exhibit good color development and gloss.

The ratio of the content of the base material to the content of the coating pigment (base material/pigment) may be 0.3 to 20, 0.5 to 15, or 1 to 10 by mass. When the ratio (base material/pigment) is within the above range, the composite pigment, when used in a cosmetic, tends to provide a smooth feel of a coating of the cosmetic, spread well when applied, and exhibit good color development and gloss.

(Coating Pigment)

The coating pigment coats the surface of the base material. The coating pigment may coat at least a part of the surface of the base material, but may completely coat the entire surface of the base material.

The coating pigment includes at least one organic pigment. The organic pigment is broadly classified into synthetic organic pigment and natural organic pigment. The coating pigment may include only one of synthetic organic pigment and natural organic pigment or may include both. The coating pigment may include different kinds of organic pigments depending on the required hue. Since the coating pigment includes an organic pigment, it is easy to obtain high color strength and vivid colors.

Examples of the synthetic organic pigment include azobased, phthalocyanine-based, anthraquinone-based, perylenebased, perinone-based, quinacridone-based, thioindigo-based, dioxazine-based, isoindolinone-based, quinophthalone-based, azomethine-based, diketopyrrolopyrrole-based, and isoindoline-based synthetic organic pigments.

Specific examples of the synthetic organic pigment include Red No. 3, Red No. 104, Red No. 106, Red No. 201, Red No. 202, Red No. 204, Red No. 205, Red No. 220, Red No. 223, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, Blue No. 404, C.I. Pigment Red 1, 2, 3, 4, 5, 6, 7, 8, 9, 12, 14, 15, 16, 17, 21, 22, 23, 31, 32, 37, 38, 41, 47, 48, 48:1, 48:2, 48:3, 48:4, 49, 49:1, 49:2, 50:1, 52:1, 52:2, 53, 53:1, 53:2, 53:3, 57, 57:1, 57:2, 58:4, 60, 63, 63:1, 63:2, 64, 64:1, 68, 69, 81, 81:1, 81:2, 81:3, 81:4, 83, 88, 90:1, 101, 101:1, 104, 108, 108:1, 109, 112, 113, 114, 122, 123, 144, 146, 147, 149, 151, 166, 168, 169, 170, 172, 173, 174, 175, 176, 177, 178, 179, 181, 184, 185, 187, 188, 190, 193, 194, 200, 202, 206, 207, 208, 209, 210, 214, 216, 220, 221, 224, 230, 231, 232, 233, 235, 236, 237, 238, 239, 242, 243, 245, 247, 249, 250, 251, 253, 254, 255, 256, 257, 258, 259, 260, 262, 263, 264, 265, 266, 267, 268, 269, 270, 271, 272, 273, 274, 275, 276, 277, 278, 279, 280, 281, 282, 283, 284, 285, 286, 287, 291, 295, 296, C.I. Pigment Blue 1, 1:2, 9, 14, 15, 15:1, 15:2, 15:3, 15:4, 15:6, 16, 17, 19, 25, 27, 28, 29, 33, 35, 36, 56, 56:1, 60, 61, 61:1, 62, 63, 66, 67, 68, 71, 72, 73, 74, 75, 76, 78, 79, C.I. Pigment Yellow 1, 2, 3, 4, 5, 10, 12, 13, 14, 15, 16, 17, 18, 24, 31, 32, 34, 35, 35:1, 36, 36:1, 37, 37:1 40, 42, 43, 53, 55, 60, 61, 62, 63, 65, 73, 74, 77, 81, 83, 93, 94, 95, 97, 98, 100, 101, 104, 106, 108, 109, 110, 113, 114, 115, 116, 117, 118, 119, 120, 123, 126, 127, 128, 129, 139, 147, 150, 151, 152, 153, 154, 155, 156, 161, 162, 164, 166, 167, 168, 169, 170, 171, 172, 173, 174, 175, 176, 177, 179, 180, 181, 182, 185, 187, 188, 192, 193, 194, 196, 198, 199, 213, 214, C.I. Pigment Violet 1, 1:1, 2, 2:2, 3, 3:1, 3:3, 5, 5:1, 14, 15, 16, 19, 23, 25, 27, 29, 31, 32, 37, 39, 42, 44, 47, 49, 50, C.I. Pigment Green 1, 2, 4, 7, 8, 10, 13, 14, 15, 17, 18, 19, 26, 36, 37, 45, 48, 50, 51, 54, 55, 58, 59. A tar dye other than those listed above can also be used as the synthetic organic pigment. These synthetic organic pigments may be used alone or in combination of two or more depending on the required hue.

Examples of the natural organic pigment (natural dye) include carotenoid-based, anthocyanin-based, flavonoidbased, quinone-based, porphyrin-based, diketone-based, betacyanin-based, and azaphilone-based natural organic pigments.

Specific examples of the natural organic pigment include beta-carotene, norbixin, bixin, capsanthin, lutein, lycopene, crocin, crocetin, astaxanthin, cyanidin acyl glucoside, cyanidin, aglycone of peonidin, cyanidin glucoside, delphinidin glucoside, anthocyanin, shisonin, malonylshisonin, pelargonidin acyl glucoside, cocoa polyphenols, apigenidin, luteolinidin, polymerized proanthocyanidins, safflomin, carthamin, carminic acid, laccaic acid, chlorophyll, phycocyanin, curcumin, betanin, isobetanin, ankaflavin, monascorubrin, iridoid glycosides, ester hydrolysates of iridoid glycosides, and eumelanin. These natural organic pigments may be used alone or in combination of two or more depending on the required hue.

The organic pigment may be a lake pigment insolubilized with metal ions. Examples of the lake pigment include aluminum lake, calcium lake, and barium lake.

The coating pigment may include a component other than the organic pigment. Examples of the component other than the organic pigment include body pigments.

The content of the organic pigment in the coating pigment may be 5% by mass or more, 10% by mass or more, 20% by mass or more, 40% by mass or more, or 60% by mass or more with respect to the total amount of the coating pigment from the viewpoint of balancing color saturation and smoothness of the composite pigment. The content of the organic pigment in the coating pigment may be 100% by mass or less, 80% by mass or less, or 60% by mass or less with respect to the total amount of the coating pigment from the viewpoint of easily keeping sparklingness of the base material. From these viewpoints, the content of the organic pigment in the coating pigment may be, for example, 5 to 100% by mass, 5 to 80% by mass, 10 to 80% by mass, 20 to 80% by mass, 40 to 60% by mass, or 60 to 100% by mass with respect to the total amount of the coating pigment. The content of the organic pigment can be determined by quantitative analysis, for

example, using high-performance liquid chromatography. Specifically, the content of the organic pigment is calculated based on a calibration curve prepared using organic pigment samples.

In a volume-based particle size distribution of the coating pigment measured by a wet laser diffraction-scattering method, when particle sizes at which cumulative values from smaller diameters reach 10% and 90% of the total volume are D10 and D90, respectively, the difference between them (D90-D10) may be 300 μm or less (0 to 300 μm). The smaller the difference (D90-D10), the less variation in particle size of the coating pigment. When D90-D10 is 300 μm or less, the composite pigment, when used in a cosmetic, tends to provide a smooth feel of a coating of the cosmetic, spread well when applied, and exhibit good color development and gloss. From the same viewpoint, the difference (D90-D10) may be 100 μm or less, 10 μm or less, or 5 μm or less. The difference (D90-D10) may be 0 μm or more, 3 μm or more, 3.5 μm or more, or 4 μm or more, and may be 0 to 300 μm, 3 to 300 μm, 3.5 to 100 μm, 4 to 10 μm, or 0 to 5 μm. D10 and D90 are measured using, for example, a particle size distribution analyzer MT3000II (MicrotracBEL Corp.).

The content of the coating pigment may be 1 to 50 parts by mass or 5 to 20 parts by mass with respect to 100 parts by mass of the base material from the viewpoint of balancing design of the base material and color saturation of the composite pigment.

(Other Components)

In addition to the base material and the coating pigment, the composite pigment may include inorganic powder or organic powder as other components.

Examples of the inorganic powder include zirconium oxide, zinc oxide, cerium oxide, magnesium oxide, barium sulfate, calcium sulfate, magnesium sulfate, calcium carbonate, magnesium carbonate, talc, sericite, silicic acid, anhydrous silicate, aluminum silicate, magnesium silicate, aluminum silicate magnesium, calcium silicate, barium silicate, strontium silicate, tungstate metal salts, hydroxyapatite, vermiculite, Higilite, bentonite, montmorillonite, hectorite, zeolite, ceramic powder, dicalcium phosphate, alumina, aluminum hydroxide, boron nitride, and silica.

Examples of the organic powder include polyamide powder, polyester powder, polyethylene powder, polypropylene powder, polystyrene powder, polyurethane powder, benzoguanamine powder, polymethylbenzoguanamine powder, tetrafluoroethylene powder, polymethyl methacrylate powder, cellulose powder, silk powder, nylon powder (nylon 12, nylon 6), styrene/acrylic acid copolymer powder, divinylbenzene/styrene copolymer powder, vinyl resin powder, urea resin powder, phenol resin powder, fluororesin powder, silicon resin powder, acrylic resin powder, melamine resin powder, epoxy resin powder, polycarbonate resin powder, microcrystalline fiber powder, rice starch, and lauroyl lysine.

When organic and inorganic powders are used, the contents of each of the organic and inorganic powders may be 0.1 to 10% by mass or 0.5 to 5% by mass with respect to the total amount of the composite pigment. The total content of the organic and inorganic powders may be in the above range.

The composite pigment may include, as other components, an oil base for the purpose of dispersion stabilization of the coating pigment. Examples of such an oil base include waxes that are solid at normal temperature (25°C) and liquid oil components.

Examples of the waxes include vegetable waxes such as candelilla wax, carnauba wax, rice wax, wood wax, and sunflower wax; animal waxes such as beeswax; mineral waxes such as ozokerite, ceresin, and microcrystalline wax; petroleum waxes such as solid paraffin; and synthetic waxes such as silicone wax and synthetic beeswax.

Examples of the liquid oil components include vegetable oils such as olive oil, castor oil, jojoba oil, macadamia nut oil, rosa canina fruit oil, cocoa butter, rose oil, and lanolin; animal oils such as horse oil, turtle oil, boar oil, mink oil, and shark oil; hydrocarbon oils such as petrolatum, fluid paraffin, isodecane, isododecane, octyldodecyl, diisostearyl malate, and hydrogenated polyisobutene; ester oils such as isotridecyl isononanoate, isopropyl isostearate, neopentyl glycol dicaprate, isotridecyl isononanoate, glyceryl diisostearate, glyceryl triisostearate, diisostearyl malate, octyldodecanol, di(phytosteryl/2-octyldodecyl) N-lauroyl-L-glutamate; silicone oils such as dimethylpolysiloxane and phenylmethylpolysiloxane; dimer acid esters, dimer diol derivatives, cholesterol fatty acid esters, phytosterol fatty acid esters, polyglycerol fatty acid esters, pentaerythritol fatty acid esters, and glyceryl tri-2-ethylhexanoate.

The total content of waxes and liquid oil components in the composite pigment may be 0.1 to 10% by mass or 0.5 to 5% by mass with respect to the total amount of the composite pigment.

The composite pigment described above can be used in cosmetics as well as inks, paints, toners, and materials for forming molded products.

<Method for Producing Composite Pigment>

Another embodiment of the present disclosure is a method for producing a composite pigment including a base material and a pigment coating a surface of the base material. The method includes a coating step of coating the surface of the base material with the pigment including an organic pigment (hereinafter referred to as "pigment for coating") by compounding the base material and the pigment for coating using a dry particle compounding device. When the amount of

load power consumed by the dry particle compounding device in the coating step is P1, the amount of load power consumed by the dry particle compounding device when the coating step is performed without a raw material is P2, and the mass of the raw material (the amount of feed) used in the coating step is m, (P1-P2)/m is 0.2 to 0.5 W·h/g.

As used herein "raw material" refers to a material used in the production of the composite particles, specifically, a mixture of the base material, the pigment for coating, and other components optionally blended. The details of the base material, the pigment for coating, and other components are the same as the details of the base material, the coating pigment, and other components in the composite pigment described above. For example, in a volume-based particle size distribution of the pigment for coating measured by a wet laser diffraction-scattering method, when particle sizes at which cumulative values from smaller diameters reach 10% and 90% of the total volume are D10 and D90, respectively, the difference between them (D90-D10) may be 300 μm or less, 100 μm or less, 10 μm or less, or 5 μm or less, may be 3 μm or more, 3.5 μm or more, 4 μm or more, and may be 0 to 300 μm, 3 to 300 μm, 3.5 to 100 μm, 4 to 10 μm, or 0 to 5 μm.

(P1-P2)/m in the above production method means the amount of load power applied per gram of the raw material in the coating step. The dry particle compounding device requires load power even during dry running. The amount of load power P2 consumed during dry running is measured in advance, and the amount of load power consumed to compound the base material and the pigment for coating is obtained by subtracting P2 from the amount of load power P1 consumed in the coating step.

Organic pigments tend to be inferior to inorganic pigments in terms of bleed resistance described above. However, in the above production method, since (P1-P2)/m is 0.2 to 0.5 W·h/g, compounding the base material and the pigment for coating proceeds well, and as a result, a composite pigment with excellent bleed resistance can be obtained. Specifically, for example, the composite pigment with an absorbance A of 0.15 or less in the above embodiment can be obtained. Therefore, it can be said that the above production method can produce a composite pigment less likely to cause pigmentation on the skin when used as a cosmetic.

(Coating Step)

In the coating step, the raw material is fed into the dry particle compounding device and treated in the device to compound the base material and the pigment for coating. The order in which the raw material is fed is not particularly limited. For example, the base material and the pigment for coating may be charged sequentially or simultaneously into the dry particle compounding device. When a premix is prepared by mixing the base material and the pigment for coating in advance, and then the premix is charged into the dry particle compounding device, it is easier to obtain higher raw material mixing performance.

The amount of raw material fed in the coating step may be such that the content of each constituent material in the resulting composite pigment is in the range indicated as the content of each constituent material in the composite pigment of the above embodiment. Specifically, for example, the amount of pigment for coating fed may be 1 to 50 parts by mass or 5 to 20 parts by mass with respect to 100 parts by mass of the base material.

As the dry compounding device in the coating step, the device described in PTL 2 above (e.g., the device described using FIGS. 1 to 3) may be used. Specific examples of the device include NOBILTA NOB ("NOBILTA" is a registered trademark) manufactured by HOSOKAWA MICRON CORPORATION and "Hybridization System NHS-O" manufactured by Nara Machinery Co., Ltd.

The dry compounding device includes, for example, a rotating section (rotor) including the rotating shaft of the device described in PTL 2 above and a stirring blade (blade) as a stirring member. The rotating section is, for example, cylindrical.

The diameter (φ) of the rotating section may be 80 mm or more (e.g., 80 to 300 mm) and may be 100 to 150 mm. Here, the diameter (φ) of the rotating section means the length of a line segment connecting an end of one stirring member to an end of another stirring member on the opposite side of the one stirring member in a virtual line passing through the center of the rotating section (the center of the rotating shaft) when viewed from the axial direction of the rotating shaft. If the length of the line segment differs depending on measurement points, the length of the longest line segment is considered as the diameter of the rotating section. The diameter of the rotating section is usually equal to twice the distance from the center of the rotating section to the farthest end of the stirring member and equal to a value obtained by subtracting twice the size of the clearance described below from the diameter (D1 in FIG. 3) of the inner circumference of the casing described below.

The wider the stirring member, the more area that can be treated. Therefore, the width of the stirring member may be 15 mm or more, 25 mm or more, or 30 mm or more. The width of the stirring member may be 15 to 60 mm, 25 to 55 mm, or 30 to 55 mm. Here, the width of the stirring member means the length from one end to the other end of the stirring member (e.g., the distance between L1 and L3 in FIG. 2) when viewed from the direction crossing the axial direction of the rotating shaft.

The number of stirring members arranged in the axial direction of the rotating shaft may be, for example, 2 or more, 2 to 10, 3 to 9, or 4 to 8. The number of stirring members arranged in the circumferential direction of the rotating shaft

when viewed from the axial direction of the rotating shaft is, for example, 4 or less, may be 1 to 4 or 2 to 3. The total number of stirring members may be, for example, 2 to 40 or 10 to 20.

The length of the casing may be 30 mm or more (e.g., 30 to 300 mm) and may be 200 to 300 mm. Here, the length of the casing means the length of the casing along the direction of the rotating shaft.

The diameter of the inner circumference of the casing (D1 in FIG. 3) may be 50 mm or more (e.g., 50 to 300 mm) and may be 100 to 200 mm.

The size of the gap (clearance) between the casing and the stirring member (minimum distance between the casing and the stirring member) may be 2 mm or more or 3 mm or more, from the viewpoint of suppressing crushing of the base material. The size of the clearance may be 10 mm or less or 6 mm or less from the viewpoint of allowing the compounding process to proceed well. From these viewpoints, the size of the clearance may be, for example, 2 to 10 mm or 3 to 6 mm.

The effective processing volume of the dry particle compounding device may be 0.015 L or more (e.g., 0.015 to 0.50 L) or 0.15 L or more (e.g., 0.15 to 0.45 L).

In the coating step, the above (P1-P2)/m is 0.2 to 0.5 W·h/g. (P1-P2)/m may be 0.3 W·h/g or more from the viewpoint of better bleed resistance of the resulting composite pigment. (P1-P2)/m may be 0.5 W·h/g or less, 0.45 W·h/g or less, or 0.4 W·h/g or less from the viewpoint of better bleed resistance of the resulting composite pigment. From the above viewpoint, (P1-P2)/m may be, for example, 0.3 to 0.5 W·h/g, 0.3 to 0.45 W·h/g, or 0.3 to 0.4 W·h/g.

(P1-P2)/m can be adjusted by the shape and number of stirring members, clearance of the device, peripheral speed of the stirring members, rotation speed of the stirring members, mixing time, and the like, in addition to the amount of raw material fed.

The peripheral speed of the stirring members in the coating step may be 5.0 to 20.0 m/s, 5.0 to 15.0 m/s, 10.0 to 15.0 m/s, or 10.0 to 13.0 m/s from the viewpoint of preventing cracking of the base material and smoothing the surface condition of the composite pigment.

The rotation speed of the stirring members in the coating step may be 500 rpm or more (e.g., 500 to 5000 rpm), and may be 1000 to 5000 rpm, 1500 to 3500 rpm, or 1500 to 2500 rpm from the viewpoint of allowing the compounding process to proceed efficiently.

The mixing time in the coating step may be 1 to 10 minutes or 2 to 10 minutes from the viewpoint of being able to suppress crushing of the base material while allowing the compounding process to proceed well.

The amount of electric power used by the dry particle compounding device in the coating step may be 100 to 2000 W.

(Other Steps)

In addition to the above coating step, the method for producing a composite pigment may further include a step of performing a process such as sterilization and sieving.

<Cosmetic, Ink, Paint, Toner, or Molded Product>

Some other embodiments of the present disclosure are a cosmetic, an ink, a paint, a toner, or a molded product containing the composite pigment of the above embodiment. These may contain general components suitable for the respective applications and purposes, in addition to the composite pigment.

Examples of the cosmetic include foundation (face color, concealer, etc.), makeup primer (make-up base, premake-up, etc.), powder (face powder), lipstick (lipstick, lip rouge, lip color, lip pencil, paste rouge, lip gloss, lip liner, etc.), eye makeup (eye shadow, eye color, eye liner, eyebrow powder, eyebrow pencil, eyebrow blush, mascara, eyelash cosmetics, etc.), cheek cosmetics (blusher, cheek color, cheek rouge, etc.), nail cosmetics (nail enamel, manicure, nail color, nail polish, toenail, nail lacquer, top coat, base coat, etc.), hair coloring (hair dye, hair color spray, hair color stick, color rinse, hair manicure, etc.).

The ink may be a printing ink. Examples of the printing ink include planographic (offset) inks, letterpress inks, intaglio (gravure) inks, stencil (screen) inks, flexographic inks, UV-curing inks, water-based inks, oil-based inks, vegetable oil inks, newspaper inks, and inkjet inks.

Examples of the paint include powder paints, water-based (aqueous) paints, and synthetic resin paints such as epoxy resin paints, urethane resin paints, fluororesin paints, polyester resin paints, melamine resin paints, and acrylic resin paints.

The toner is a micro-sized powder used in laser printers and copiers, including colored particles attached to electrically chargeable plastic particles. In the toner, the composite pigment may be blended as the colored particles.

The molded product may be molded using a resin composition containing the composite pigment as a plastic colorant. The plastic colorant can be in any of the following forms: masterbatch (MB), colored pellet/colored compound, dry color, and paste color/liquid masterbatch.

[EXAMPLES]

The present disclosure will be described in more detail below with examples and comparative examples, but the present disclosure is not limited to the examples below.

<Materials and Devices Used>

In examples and comparative examples, the following base materials 1 to 5 and pigments (pigments for coating) 1 to 5, and devices (dry compounding devices) 1 to 2 were used.

[Base Material]

- Base material 1
  Mica particles coated with titanium oxide (red, flaky, manufactured by Sun Chemical, D50: 23 μm)
- Base material 2
  Mica particles coated with titanium oxide (red, flaky, manufactured by Sun Chemical, D50: 20 μm)
- Base material 3
  Mica particles coated with titanium oxide (gold, flaky, manufactured by Sun Chemical, D50: 23 μm)
- Base material 4
  Mica particles coated with titanium oxide (green, flaky, manufactured by Sun Chemical, D50: 23 μm)
- Base material 5
  Mica particles coated with titanium oxide (silver, flaky, Colors & Effects, D50: 40 μm)

[Pigment for Coating]

- Pigment 1
  Pigment (C19-7703, manufactured by Sun Chemical, organic pigment content: 60% by mass, D90-D10: 3.90 μm) including an organic pigment (Red No. 202, azo-based, C.I. Pigment Red 57:1)
- Pigment 2
  Pigment (C19-7720, manufactured by Sun Chemical, organic pigment content: 100% by mass, D90-D10: 3.28 μm) made of an organic pigment (Red No. 202, azo-based, C.I. Pigment Red 57:1)
- Pigment 3
  Pigment (C19-003, manufactured by Sun Chemical, organic pigment content: 60% by mass, D90-D10: 87.06 μm) including an organic pigment (Red No. 202, azo-based, C.I. Pigment Red 57:1)
- Pigment 4
  Pigment (C14-7723, manufactured by Sun Chemical, organic pigment content: 22% by mass, D90-D10: 6.59 μm) including an organic pigment (Red No. 104 aluminum lake, tar dye)
- Pigment 5
  Pigment (C39-7733, manufactured by Sun Chemical, organic pigment content: 17% by mass, D90-D10: 4.76 μm) including an organic pigment (Blue No. 1 aluminum lake, tar dye)

[Dry Compounding Device]

- Device 1 (NOBILTA (registered trademark) NOB-130)

  Blade diameter (φ) : 124 mm
  Blade width: 49 mm
  Number of blades: 16 in total (number of blades arranged in the axial direction: 6, number of blades arranged in the circumferential direction: 2)
  Casing length: 230 mm
  Inner diameter of casing: 130 mm
  Clearance: 3 mm

Effective processing volume: 0.269 L

- Device 2 (NOBILTA (registered trademark) NOB-mini)

Blade diameter (φ): 86mm
Blade width: 20mm
Number of blades: 8 in total (number of blades arranged in the axial direction: 2, number of blades arranged in the circumferential direction: 4)
Casing length: 60mm
Inner diameter of casing: 92mm
Clearance: 2mm
Effective processing volume: 0.033 L

<Examples 1 to 7 and Comparative Example 1>

(Production of Composite Pigment)

The base material and the pigment for coating were mixed in advance in the amounts listed in Table 1 to prepare a premix, and the premix was charged into the dry particle compounding device (device 1 or 2) listed in Table 1 and treated under the conditions (amount of electric power, mixing time, amount of load power, rotation speed, peripheral speed) listed in Table 1 to obtain a composite pigment. The amount of load power in Table 1 is the amount of load power applied per gram of the raw material (the base material and the pigment for coating), and corresponds to the above (P1-P2)/m. The rotation speed and the peripheral speed in Table 1 are the rotation speed and the peripheral speed of the blades.

(Measurement of Absorbance A)

The absorbance A described above was measured for the composite pigments of Examples 1 to 7 and Comparative Example 1. Specifically, 0.05 g of the composite pigment was dispersed in 20 mL of ethyl acetate, and the resulting dispersion was allowed to stand for 24 hours and separated into a precipitate and a supernatant. The absorbance of the supernatant was measured in the wavelength range of 300 to 800 nm using a spectrophotometer U-3900 manufactured by Hitachi, Ltd., and the absorbance at the maximum absorption wavelength (absorbance A) was determined. The results are listed in Table 1. The maximum absorption wavelength near 515 nm was read for Examples 1 to 4 and 7 and Comparative Example 1, the maximum absorption wavelength near 560 nm was read for Example 5, and the maximum absorption wavelength near 670 nm was read for Example 6.

(Measurement of Strong Coverage Ratio)

The strong coverage ratio was measured for the composite pigments of Examples 1 to 7 and Comparative Example 1. Specifically, first, absorbance A1 and absorbance A2 were measured according to conditions 1 and 2 described above, and the strong coverage ratio was calculated based on the above formula (a). The results are listed in Table 1. Ethanol (acidic dilution) was used as a diluting solvent in conditions 1 and 2 in Examples 1 to 4 and 7 and in Comparative Example 1, and a sodium hydroxide reagent (dilution) was used in Examples 5 and 6. The maximum absorption wavelength near 521 nm was read for Examples 1 to 4 and 7 and Comparative Example 1, the maximum absorption wavelength near 538 nm was read for Example 5, and the maximum absorption wavelength near 630 nm was read for Example 6.

<Evaluation>

The pigmentation resistance was evaluated for the composite pigments of Examples 1 to 7 and Comparative Example 1. Specifically, first, the composite pigment (about 50 mg) was applied and spread with a finger on a 5 mm × 5 mm or larger section of a BIOSKIN plate 100 × 100 mm urethane No. 132 series (No.132#W manufactured by Beaulax Co., Ltd.). Then, excess pigment was lightly blown off with air, and an appropriate amount (about 0.1 g) of cleansing oil (Deve OLIVE & ARGAN manufactured by BEAUA PRODUCTS Co., Ltd.) was put on the pigment-applied area. The pigment-applied area was then rubbed with a finger for two seconds, the oil was wiped off with KimWipes, and the pigment-applied area was wiped up and wiped off. After wiping up, the pigment-applied area was colorimetrically measured with a colorimeter (eXact Advanced manufactured by x-Rite) to obtain the C* value. The colorimetric conditions were illuminant: D50 and standard observer: 2°. The results are listed in Table 1.

[Table 1]

| | Base material | | | Pigment for coating | | | Device/Conditions | | | | | | Composite pigment | | Evaluation |
|---|---|---|---|---|---|---|---|---|---|---|---|---|---|---|---|
| | Type | D50 | Amount fed | Type | D90 - D10 | Amount fed | Device | Amount of electric power | Mixing time | Amount of load power | Rotation speed | Peripheral speed | Absorbance A | Strong coverage ratio | Color pigmentation (C* value) |
| | [-] | [μm] | [g] | [-] | [μm] | [g] | [-] | [w] | [min] | [w·h/g] | [rpm] | [m/s] | [-] | [%] | [-] |
| Ex. 1 | Base material 1 | 23 | 340 | Pigment 1 | 3.9 0 | 38 | 1 | 964 | 5 | 0.21 3 | 1800 | 11.7 | 0.056 | 88.4% | 1.86 |
| Ex. 2 | Base material 2 | 20 | 36 | Pigment 1 | 3.9 0 | 4 | 2 | 188 | 3.1 | 0.24 3 | 3000 | 13.5 | 0.055 | 87.7% | 1.88 |
| Ex. 3 | Base material 2 | 20 | 36 | Pigment 2 | 3.2 8 | 4 | 2 | 180 | 3.3 | 0.24 8 | 3000 | 13.5 | 0.103 | 87.4% | 1.72 |
| Ex. 4 | Base material 2 | 20 | 36 | Pigment 3 | 87. 06 | 4 | 2 | 198 | 3 | 0.248 | 3000 | 13.5 | 0.095 | 92.6% | 1.76 |
| Ex. 5 | Base material 3 | 23 | 331 | Pigment 4 | 6.5 9 | 37 | 1 | 1715 | 5 | 0.388 | 2080 | 11.7 | 0.020 | 95.0% | 1.41 |
| Ex. 6 | Base material 4 | 23 | 334 | Pigment 5 | 4.7 6 | 37 | 1 | 1379 | 7 | 0.434 | 2080 | 11.7 | 0.011 | 94.8% | 1.72 |
| Ex. 7 | Base material 5 | 40 | 477 | Pigment 1 | 3.9 0 | 53 | 1 | 1400 | 5 | 0.220 | 1550 | 10.1 | 0.141 | 84.1% | 0.98 |
| Com p. Ex. 1 | Base material 2 | 20 | 36 | Pigment 2 | 3.2 8 | 4 | 2 | 129 | 3 | 0.161 | 3000 | 13.5 | 0.172 | 86.3% | 3.51 |

## Claims

1. A composite pigment including a base material and a pigment coating a surface of the base material, wherein

   the pigment includes an organic pigment, and
   when 0.05 g of the composite pigment is dispersed in 20 mL of ethyl acetate and a resulting dispersion is allowed to stand for 24 hours and separated into a precipitate and a supernatant, the supernatant has an absorbance of 0.15 or less at a maximum absorption wavelength.

2. The composite pigment according to claim 1, wherein the composite pigment has a strong coverage ratio of 80% or more.

3. The composite pigment according to claim 1 or 2,
   wherein the base material and the pigment are compounded by mechanochemical treatment.

4. The composite pigment according to claim 1 or 2,
   wherein the base material is a flaky base material including at least one selected from the group consisting of mica, aluminum, alumina, and glass.

5. The composite pigment according to claim 1 or 2,
   wherein

   the base material includes a core and a shell coating a surface of the core, and
   the shell includes at least one selected from the group consisting of resins, metals, and metal oxides.

6. A method for producing a composite pigment including a base material and a pigment coating a surface of the base material,

   the method including a coating step of coating the surface of the base material with the pigment by compounding the base material and the pigment including an organic pigment using a dry particle compounding device, wherein when the amount of load power consumed by the dry particle compounding device in the coating step is P1, the amount of load power consumed by the dry particle compounding device when the coating step is performed without a raw material is P2, and the mass of the raw material used in the coating step is m, (P1-P2)/m is 0.2 to 0.5 W·h/g.

7. The method for producing a composite pigment according to claim 6, wherein in a volume-based particle size distribution of the pigment measured by a wet laser diffraction-scattering method, when particle sizes at which cumulative values from smaller diameters reach 10% and 90% of a total volume are D10 and D90, respectively, D90-D10 is 300 μm or less.

FIG. 1

4
1
6
2
3
9
5
7
8

FIG. 2

3
3a(1)
3a(3)
3a(5)
3b(2)
3b(4)
3b(6)
2a
2a
10
10
L1
L3
RETURN DIRECTION
FEED DIRECTION

FIG. 3

5
1
3a(5)
4
2
D1
9
3b(6)
3b(6)
D2
10
3a(5)

## INTERNATIONAL SEARCH REPORT

International application No. **PCT/JP2024/042193**

**A. CLASSIFICATION OF SUBJECT MATTER**

***C09B 67/02***(2006.01)i; ***A61K 8/25***(2006.01)i; ***A61K 8/26***(2006.01)i; ***A61Q 1/00***(2006.01)i
FI: C09B67/02 A; A61K8/25; A61K8/26; A61Q1/00

According to International Patent Classification (IPC) or to both national classification and IPC

**B. FIELDS SEARCHED**

Minimum documentation searched (classification system followed by classification symbols)

C09B67/02; A61K8/25; A61K8/26; A61Q1/00

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 2023-109890 A (DIC CORPORATION) 08 August 2023 (2023-08-08)<br>claims 1-4, paragraphs [0007], [0010], [0013]-[0039], examples 1-8 | 1-7<br><br>5 |
| X<br><br>Y | JP 7-331113 A (DAINICHISEIKA COLOR & CHEMICALS MFG. CO., LTD.) 19 December 1995 (1995-12-19)<br>claims 1, 3-6, paragraphs [0011]-[0018], examples 1-2 | 1-7<br><br>5 |
| X<br><br>Y | JP 8-59434 A (KOSE CORP.) 05 March 1996 (1996-03-05)<br>claims 1-2, paragraphs [0001]-[0011], examples 1-7 | 1-4, 6-7<br><br>5 |
| X | JP 2006-176742 A (NIPPON SHEET GLASS COMPANY, LIMITED) 06 July 2006 (2006-07-06)<br>claims 1-7, paragraphs [0027]-[0059], examples 1-4, comparative examples 2-3 | 1-2, 4-5 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 February 2025** | **18 February 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

INTERNATIONAL SEARCH REPORT

International application No.
**PCT/JP2024/042193**

**C. DOCUMENTS CONSIDERED TO BE RELEVANT**

| Category* | Citation of document, with indication, where appropriate, of the relevant passages | Relevant to claim No. |
|---|---|---|
| X | JP 62-101662 A (MERCK PATENT GMBH) 12 May 1987 (1987-05-12) claims, examples 1-13 | 1-2, 4-5 |
| X | JP 2002-356625 A (TODA KOGYO CORP.) 13 December 2002 (2002-12-13) claims 1-2, paragraphs [0024]-[0074], examples | 1-2, 4-5 |
| X | JP 2017-528555 A (MERCK PATENT GMBH) 28 September 2017 (2017-09-28) claims 1-27, paragraphs [0011]-[0085], examples 1-2 | 1-2, 4-5 |
| X | JP 05-214257 A (MERCK JAPAN KK) 24 August 1993 (1993-08-24) claims 1-5, paragraphs [0002], [0008]-[0027], examples 1-8 | 1-7 |

Form PCT/ISA/210 (second sheet) (July 2022)

## INTERNATIONAL SEARCH REPORT

International application No.
**PCT/JP2024/042193**

**Box No. III Observations where unity of invention is lacking (Continuation of item 3 of first sheet)**

This International Searching Authority found multiple inventions in this international application, as follows:

Claims 1–5 lack novelty in light of documents 1–8 and thus do not have the special technical feature.
Thus, the invention in claims 1–5 is classified as invention 1.
Claims 6–7 share, with claim 1, the common technical feature of a "composite pigment comprising: a substrate; and a pigment coating a surface of the substrate, wherein the pigment includes an organic pigment."
However, the technical feature does not make a contribution over the prior art in light of documents 1–8.
Thus, claims 6–7 are classified as invention 2.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/042193**

| Patent document cited in search report | Publication date (day/month/year) | Patent family member(s) | Publication date (day/month/year) |
|---|---|---|---|
| JP 2023-109890 A | 08 August 2023 | US 2024/0101831 A1<br>claims 1-4, paragraphs [0023]-[0051], examples 1-8<br>WO 2022/176336 A1<br>EP 4296319 A1<br>CN 116685645 A<br>KR 10-2023-0146003 A | |
| JP 7-331113 A | 19 December 1995 | (Family: none) | |
| JP 8-59434 A | 05 March 1996 | (Family: none) | |
| JP 2006-176742 A | 06 July 2006 | US 2007/0134179 A1<br>claims 1-7, paragraphs [0035]-[0072], examples, comparative examples<br>WO 2005/071019 A1<br>EP 1707599 A1<br>KR 10-2006-0132643 A<br>CN 1910243 A | |
| JP 62-101662 A | 12 May 1987 | US 4772331 A<br>claim 1, examples 1-23<br>EP 220617 A2<br>KR 10-1987-0004112 A | |
| JP 2002-356625 A | 13 December 2002 | US 2003/0116758 A1<br>claims 11-12, paragraphs [0049]-[0111], examples<br>EP 1184426 A2<br>KR 10-2002-0025673 A<br>CN 1342731 A | |
| JP 2017-528555 A | 28 September 2017 | US 2017/0210905 A1<br>claims 1-27, paragraphs [0035]-[0142], examples 1-2<br>WO 2016/012074 A1<br>EP 3172281 A1<br>TW 201619299 A | |
| JP 05-214257 A | 24 August 1993 | US 5336309 A<br>claims 1-19, examples 1-8<br>EP 554776 A1<br>KR 10-1993-0017984 A<br>TW 261630 B | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H05214257 A
- JP 2005270955 A